# EUROPÄISCHE PATENTSCHRIFT

(11) **EP 2 872 465 B1**
(45) Veröffentlichungstag und Bekanntmachung des Hinweises auf die Patenterteilung: **20.12.2017**
(21) Anmeldenummer: 13730489.5
(22) Anmeldetag: 18.06.2013
(51) Int. Cl.: C07C 1/24, C07C 45/50, C07C 51/235, C07C 67/08, C10M 105/32

(54) **Verfahren zur Herstellung von Carbonsäureestern eines Gemisches aus strukturverzweigten C9-Monocarbonsäuren ausgehend von 2-Ethylhexanol, Carbonsäureester des Triethylenglykols, Neopentylglykols und 1,3-Butandiols dieses Gemisches, sowie ihre Verwendung**
Process for the preparation of carboxylic acid esters of a mixture of structually branched C9-monocarboxylic acids starting from 2-ethylhexanol, carboxylic acid esters of triethylene glycol, neopentyl glycol and 1,3-butanediol of this mixture as well as their use
Procédé pour la préparation des esters d'acides carboxyliques d'une mélange des acides monocarboxyliques en C9 structurellement ramifiés à partir de 2-éthylhexanol, esters d'acides carboxyliques de triéthylène glycol, néopentyl glycol et 1,3-butanediol de cette mélange et leur utilisation

(30) Priorität: 13.07.2012 DE 102012013968
(43) Veröffentlichungstag der Anmeldung: 20.05.2015
(73) Patentinhaber: OXEA GmbH, 46147 Oberhausen (DE)
(72) Erfinder: FREY, Guido D., 64560 Riedstadt (DE); EISENACHER, Matthias, 46485 Wesel (DE); KOCKRICK, Kristina, 40505 Düsseldorf (DE); STRUTZ, Heinz, 47445 Moers (DE)
(86) Internationale Anmeldenummer: PCT/EP2013/001797
(87) Internationale Veröffentlichungsnummer: WO 2014/008974

(56) Entgegenhaltungen:
- EP-A2- 2 308 821
- WO-A1-03/029180
- DE-A1- 2 604 545
- DE-A1- 19 908 320

## Beschreibung

Die vorliegende Erfindung betrifft die Herstellung von Carbonsäureestern eines Gemisches aus strukturverzweigten C9-Monocarbonsäuren durch Dehydratisierung von 2-Ethylhexanol, Hydroformylierung des erhaltenen Octengemisches zu einem Gemisch isomerer Isononanale, nachfolgende Oxidation zu den entsprechenden Isononansäuren und Veresterung mit Alkoholen, Carbonsäureester des Triethylenglykols, Neopentylglykols und 1,3-Butandiols dieses Gemisches, sowie ihre Verwendung.

Isononansäure, ein Gemisch aus strukturverzweigten C9-Monocarbonsäuren, ist ein wichtiges Zwischenprodukt in der industriellen organischen Chemie, das zu einer Vielzahl von Folgeprodukten für verschiedenste Anwendungsgebiete verarbeitet wird. Beispielsweise werden ihre Salze als Trocknungsbeschleuniger oder Sikkative für Lacke verwendet.

Isononansäure wird in großen Mengen zu Carbonsäureestern weiterverarbeitet, die als Schmiermittel Verwendung finden. Insbesondere die Veresterung mit mehrwertigen Alkoholen wie Neopentylglykol, Trimethylolpropan, Di-Trimethylolpropan, Pentaerythrit oder Di-Pentaerythrit ergibt Schmiermittel, die beim Betrieb von Kältemaschinen eingesetzt werden. Isononansäure wird dabei häufig im Gemisch mit anderen C₄-C₁₂-Monocarbonsäuren wie 2-Methylbuttersäure, n-Pentansäure, n-Heptansäure, 2-Ethylhexansäure oder n-Octansäure verestert (EP 1 281 701 A1, EP 1 199 300 A2, EP 0 903 335 A1, EP 0 475 751 A1, WO 90/12849 A1).

Ester der Isononansäure werden ebenfalls als Weichmacher für thermoplastische Kunststoffe eingesetzt. Weichmacher für PVC auf Basis von Iso-nonansäure mit Polyolen werden beispielsweise in WO 95/19389 A1 beschrieben. Eine spezielle Klasse von Esterweichmachern, die auch mit der Bezeichnung G-Ester abgekürzt werden, enthält als Alkoholkomponente Etherdiole wie Diethylenglykol, Triethylenglykol oder Tetraethylenglykol. Sie werden zur Weichmachung von Polyvinylbutyralfolien benutzt, die als Zwischenschicht bei der Herstellung von Mehrschichten- oder Verbundgläsern verwendet werden. Sie können ebenfalls als Koaleszenzmittel oder Filmbildehilfsmittel in wässrigen Dispersionen von Kunststoffen, die als Beschichtungsmittel vielfältige Anwendung finden, eingesetzt werden (DE 10 2009 048 771 A1, DE 199 40 991 A1). Nach EP 2 308 821 A2 wird das rohe Veresterungsprodukt zur Farbaufhellung mit Ozon und ozonhaltigen Gasen behandelt und unmittelbar anschließend einer Wasserdampfbehandlung unterzogen.

Für die genannten Anwendungen kommt überwiegend eine Isononansäure zum Einsatz, die die strukturisomere Form 3,5,5-Trimethylhexansäure als Hauptbestandteil enthält. Das C-9 Kohlenwasserstoffgerüst 3,5,5-Trimethylhexyl basiert auf dem petrochemischen Vorprodukt Isobuten, das in Gegenwart saurer Katalysatoren zu Diisobuten dimerisiert wird und von den dabei ebenfalls gebildeten höheren Oligomerisaten destillativ abgetrennt wird (Hydrocarbon Processing, April 1973, Seiten 171-173; Ullmanns Encyclopedia of Industrial Chemistry, 6th. Ed., 2003, Vol. 6, Seite 3). Diisobuten besteht im Wesentlichen aus den isomeren Octenen 2,4,4-Trimethyl-1-penten und 2,4,4-Trimethyl-2-penten und kann durch Oxo-Reaktion oder Hydroformylierungsreaktion mit Kohlenmonoxid und Wasserstoff in Gegenwart von Rhodium- oder Kobaltkatalysatoren in den entsprechenden Aldehyd 3,5,5-Trimethylhexanal umgewandelt werden (Ullmann's Encyclopedia of Industrial Chemistry, 6th. Ed., 2003, Vol. 2, Seite 68, 75; DE 2737633 A). Weitere C9-Isomere, die in geringen Mengen zugegen sind, sind 3,4,4- und 3,4,5-Trimethylhexanal sowie 2,5,5-Trimethylhexanal, 4,5,5-Trimethylhexanal und 6,6-Dimethylheptanal. Durch Oxidation dieses Aldehydgemisches erhält man eine technisch verfügbare Isononansäure, die üblicherweise einen Gehalt an 3,5,5-Trimethylhexansäure von etwa 90% aufweist (Ullmanns Encyklopädie der technischen Chemie, 4. Auflage, 1975, Verlag Chemie, Band 9, Seiten 143-145; EP 1 854 778 A1). Gemäß DE 199 08 320 A1 wird Di-Buten, das aus einem Butenoligomerisat abgetrennt wird, durch die Hydrocarboxylierung oder durch die Hydroformylierung mit anschließender Oxidation in ein Gemisch isomerer C9-Monocarbonsäuren überführt, das anschließend in Vinylester umgewandelt wird. Diese Vinylester können als Weichmacher verwendet werden.

Die bedeutendste Rohstoffquelle für Isobuten ist der C4-Schnitt aus der Dampfspaltung von Naphtha. Seine Verfügbarkeit im Vergleich zu den C2- und C3-Spaltprodukten kann durch die Bedingungen der Dampfspaltung gesteuert werden und richtet sich nach den Marktgegebenheiten. Aus den C4-Spaltprodukten wird zunächst 1,3-Butadien durch Extraktion oder durch Selektivhydrierung in n-Butene entfernt. Das erhaltene C4-Raffinat, auch als Raffinat I bezeichnet, enthält überwiegend die ungesättigten Butene Isobuten, 1-Buten und 2-Buten sowie die hydrierten Produkte n-Butan und Isobutan. Aus dem Raffinat I wird im nächsten Schritt Isobuten entfernt und das erhaltene, isobutenfreie C4-Gemisch bezeichnet man als Raffinat II.

Für die Isobutenabtrennung werden in der industriellen Produktion verschiedene Verfahren angewandt, bei denen man die relativ höchste Reaktivität des Isobutens in dem Raffinat I ausnutzt. Bekannt ist die reversible protonenkatalysierte Wasseranlagerung zum tertiär-Butanol oder die Methanolanlagerung zum Methyl-tertiär-butylether. Aus diesen Additionsprodukten kann durch Rückspaltung wieder Isobuten zurückgewonnen werden (Weissermel, Arpe, Industrielle Organische Chemie, VCH Verlagsgesellschaft, 3. Auflage, 1988, S. 74-79).

Ebenfalls kann das butadienfreie C4-Raffinat bei erhöhter Temperatur und unter Druck mit einem sauren suspendierten Ionenaustauscher in Kontakt gebracht werden. Isobuten oligomerisiert zu Di-isobuten, Tri-isobuten und in geringem Maße zu höheren Oligomeren. Die Oligomeren werden von den nicht reagierten C4-Verbindungen abgetrennt. Aus dem Oligomerisat kann dann Di-isobuten oder Tri-isobuten destillativ rein gewonnen werden. Durch die Dimerisierung von n-Butenen mit Isobuten wird in geringem Maße das Codimer gebildet (Weissermel, Arpe, Industrielle Organische Chemie, VCH Verlagsgesellschaft, 3. Auflage, 1988, S. 77; Hydrocarbon Processing, April 1973, S. 171-173).

Vor dem Hintergrund, dass die Verfügbarkeit an Octenen basierend auf dem C4-Schnitt aus der Naphthaspaltung beschränkt ist und von den lokalen Standortbedingungen abhängt, ist es wünschenswert, weitere Octenquellen auf Basis preiswert verfügbarer Großprodukte zu erschließen, die auf einfache Weise zu verschiedenen Standorten transportiert werden können. 2-Ethylhexanol steht als industrielles Großprodukt preiswert zur Verfügung, das ohne Probleme weitläufig vertrieben werden kann. 2-Ethylhexanol wird bekanntermaßen durch Hydroformylierung oder Oxo-Reaktion von Propylen zu n-Butyraldehyd mit nachfolgender alkalisch katalysierter Aldolkondensation zum 2-Ethylhexenal und anschließender Vollhydrierung zum 2-Ethylhexanol großtechnisch hergestellt (Ullmann's Encyclopedia of Industrial Chemistry, 7. Auflage, 2011, Wiley, Band 13, Seiten 579-584).

Auf die Verwendung von 2-Ethylhexanol zur Herstellung eines Octengemisches, das über die Dehydratisierung, Hydroformylierung und Hydrierung zu einem Isononanolgemisch verarbeitet wird, geht WO 03/029180 A1 kurz ein. Dabei steht die Einstellung der Viskosität der isomeren Phthalsäure-dialkylester im Mittelpunkt, die durch Veresterung von isomeren Nonanolen mit Phthalsäure oder Phthalsäureanhydrid erhalten werden. Hinweise, die Dehydratisierungsprodukte von 2-Ethylhexanol in Isononansäure oder Isononansäureester zu überführen, werden nicht gegeben.

Die Nutzung von 2-Ethylhexanol als Octenquelle ermöglicht die Bereitstellung von Isononansäure auf Basis von Propylen und mindert die Abhängigkeit von der Octenverfügbarkeit auf Butenbasis. Eine auf diese Weise hergestellte Isononansäure kann dann zu Carbonsäureestern weiterverarbeitet werden.

Die vorliegende Erfindung besteht daher in einem Verfahren zur Herstellung von Carbonsäureestern eines Gemisches aus strukturverzweigten C9-Monocarbonsäuren ausgehend von 2-Ethylhexanol. Das Verfahren ist dadurch gekennzeichnet, dass man
(a) 2-Ethylhexanol in Gegenwart eines Katalysators zu einem Octengemisch dehydratisiert;
(b) das nach Schritt a) erhaltene Octengemisch in Gegenwart einer Übergangsmetallverbindung der Gruppe VIII des Periodensystems der Elemente mit Kohlenmonoxid und Wasserstoff zu einem Gemisch isomerer Isononanale umsetzt;
(c) das nach Schritt b) erhaltene Gemisch isomerer Isononanale zu einem Gemisch aus strukturverzweigten C9-Monocarbonsäuren oxidiert; und
(d) das nach Schritt c) erhaltene Gemisch aus strukturverzweigten C9-Monocarbonsäuren mit Alkoholen zu Carbonsäureestern umsetzt.

Die vorliegende Erfindung betrifft ebenfalls Carbonsäureester des Triethylenglykols, Neopentylglykols oder 1,3-Butandiols und eines Gemisches aus strukturverzweigten C9-Monocarbonsäuren enthaltend 4-Methyloctansäure, 6-Methyloctansäure, 2,5-Dimethylheptansäure, 2,3-Dimethylheptansäure, 3-Ethylheptansäure, 2-Ethylheptansäure und 2-Ethyl-4-methylhexansäure in Summe mindestens 80 mol-%, bezogen auf den Gesamtgehalt an strukturverzweigten C9-Monocarbonsäuren.

Die Dehydratisierung von 2-Ethylhexanol kann sowohl in der Flüssigphase als auch in der Gasphase an einem dafür geeigneten Katalysator durchgeführt werden. Bevorzugt erfolgt die Dehydratisierung in der Gasphase bei Temperaturen im Bereich von 200 bis 450°C, vorzugsweise von 250 bis 380°C unter Verwendung fachüblicher Reaktoren in Gegenwart heterogener Katalysatoren mit dehydratisierenden Eigenschaften, wie Aluminiumoxid in seinen verschiedenen Modifikationen, Nickel niedergeschlagen auf Aluminiumoxid, oder Phosphorsäure niedergeschlagen auf Siliziumdioxid oder Aluminiumoxid. Solche zur Dehydratisierung geeignete Heterogenkatalysatoren sind aus dem Stand der Technik her bekannt (GB 313426, US 2468764, US 2919973) und stehen beispielsweise als Al3996 der Firma BASF SE kommerziell zur Verfügung. US 2919973 behandelt die Dehydratisierung von 2-Ethylhexanol an einem heterogenen Aluminiumoxidkatalysator bei Temperaturen um 350°C und bei einer Katalysatorbelastung von 2,4 bis 2,8 Liter 2-Ethylhexanol pro Liter Katalysator und Stunde. Der Stand der Technik gibt jedoch keine Auskunft über die Isomerenverteilung in dem erhaltenen Octengemisch.

Der in dem erfindungsgemäßen Verfahren für die Dehydratisierung von 2-Ethylhexanol eingesetzte Reaktor kann neben der Katalysatorschüttung noch weitere Füllkörper oder Einbauten enthalten, beispielsweise Raschigringe, Sättel, Pallringe, Filterplatten oder Kolonnenböden. Verwendet man Füllkörper, dann werden sie vorzugsweise oberhalb der Katalysatorschüttung angebracht, um das Totvolumen zu verringern. Wird in der Flüssigphase dehydratisiert, kann auf Rührvorrichtungen, Einbauten und Füllkörper verzichtet werden, so dass in dem Reaktionsgefäß nur der Dehydratisierungskatalysator anwesend ist.

In der bevorzugten Arbeitsweise wird 2-Ethylhexanol in einem vorgeschalteten Verdampfer erhitzt und gasförmig über die Katalysatorschüttung geführt, gegebenenfalls unter Verwendung eines inerten Trägergases wie Stickstoff, Kohlendioxid oder Edelgase. Die Belastung V/Vh des heterogenen Katalysators kann über einen weiten Bereich variieren und beträgt im Allgemeinen von 0,2 bis 3,5 Liter 2-Ethylhexanol pro Liter Katalysator und Stunde. Das der Dehydratisierungszone entnommene Reaktionsgemisch wird anschließend kondensiert. Durch das abgespaltene Wasser fällt eine wässrige Phase an, die von der organischen Olefinphase durch einfache Phasentrennung separiert wird. Bei dem erhaltenen Octen handelt es sich um ein Gemisch strukturisomerer Octene mit den einfach verzweigten Octenen 2-Ethyl-1-hexen sowie cis/trans 3-Methyl-3-hepten und cis/trans 3-Methyl-2-hepten als Hauptkomponenten. Nennenswerte Mengen an Di-C8-Ethem werden nicht gebildet.

Das nach Entfernen des Spaltwassers vorliegende Octen wird anschließend ohne weitere Reinigung oder zweckmäßigerweise nach destillativer Aufreinigung für die Umsetzung mit Kohlenmonoxid und Wasserstoff in der Hydroformylierungsreaktion oder Oxo-Reaktion verwendet. Die eingesetzte Mischung aus Kohlenmonoxid und Wasserstoff bezeichnet man auch als Synthesegas. Man führt die Hydroformylierungsreaktion in einem homogenen Reaktionssystem durch. Der Begriff homogenes Reaktionssystem steht für eine im Wesentlichen aus Lösungsmittel, falls zugesetzt, Katalysator, olefinisch ungesättigter Verbindung und Reaktionsprodukt zusammengesetzte homogene Lösung. Als besonders wirksame Lösungsmittel haben sich die höher siedenden Kondensationsverbindungen der herzustellenden Aldehyde, insbesondere die Trimeren der herzustellenden Aldehyde, erwiesen, die als Nebenprodukte bei der Hydroformylierung anfallen, sowie ihre Mischungen mit dem herzustellenden Isononanal, so dass ein weiterer Lösungsmittelzusatz nicht unbedingt erforderlich ist. In einigen Fällen kann sich jedoch ein Lösungsmittelzusatz als zweckmäßig erweisen. Als Lösungsmittel werden organische Verbindungen eingesetzt, in denen Ausgangsmaterial, Reaktionsprodukt und Katalysator löslich sind. Beispiele für solche Verbindungen sind aromatische Kohlenwasserstoffe, wie Benzol und Toluol oder die isomeren Xylole und Mesitylen. Andere gebräuchliche Lösungsmittel sind Paraffinöl, Cyclohexan, n-Hexan, n-Heptan oder n-Octan, Ether wie Tetrahydrofuran, Ketone oder Texanol® der Firma Eastman. Der Anteil des Lösungsmittels im Reaktionsmedium kann über einen weiten Bereich variiert werden und beträgt üblicherweise zwischen 20 und 90 Gew.-%, vorzugsweise 50 bis 80 Gew.-%, bezogen auf das Reaktionsgemisch. Die Hydroformylierung des Octens kann aber auch ohne Lösungsmittelzusatz erfolgen. Die Hydroformylierungsreaktion wird typischerweise in homogener organischer Phase in Gegenwart mindestens einer Übergangsmetallverbindung der Gruppe VIII des Periodensystems der Elemente durchgeführt. Die Umsetzung kann sowohl in Gegenwart sowie in Abwesenheit von komplexbildenden Organoelementverbindungen, die als Komplexliganden wirken, durchgeführt werden.

Wird die Hydroformylierungsreaktion in Gegenwart von Komplexliganden durchgeführt, eignet sich die Verwendung von Organophosphorverbindungen als Organoelementverbindungen. Derartige Komplexverbindungen und ihre Herstellung sind bekannt (US 3 527 809 A, US 4 148 830 A, US 4 247 486 A, US 4 283 562 A). Sie können als einheitliche Komplexverbindungen oder auch als Gemisch unterschiedlicher Komplexverbindungen eingesetzt werden. Die Übergangsmetallkonzentration im Reaktionsmedium erstreckt sich über einen breiten Bereich von etwa 1 bis etwa 1000 Gew.-ppm und beträgt vorzugsweise 10 bis 700 Gew.-ppm und insbesondere 25 bis 500 Gew.-ppm, jeweils bezogen auf das homogene Reaktionsgemisch. Als Katalysator kann die stöchiometrisch zusammengesetzte Übergangsmetall-Komplexverbindung Anwendung finden. Es hat sich jedoch als zweckmäßig erwiesen, die Hydroformylierung in Gegenwart eines Katalysatorsystems aus Übergangsmetall-Komplexverbindung und freiem Komplexliganden durchzuführen, der mit dem Übergangsmetall keine Komplexverbindung mehr eingeht. Der freie Komplexligand kann der Gleiche sein wie in der Übergangsmetall-Komplexverbindung, es können aber auch von diesem verschiedene Komplexliganden eingesetzt werden. Zu den bevorzugten Komplexliganden zählen Triarylphosphine wie Triphenylphosphin, Trialkylphosphine wie Tri(cyclohexyl)phosphin, Alkylphenylphosphine, organische Phosphite oder Diphosphite. Das molare Verhältnis von Übergangsmetall zu Komplexligand beträgt im Allgemeinen 1:1 bis 1:1000, es kann aber auch noch höher liegen. Bevorzugt setzt man das Übergangsmetall und den Komplexliganden in einem molaren Verhältnis von 1:3 bis 1:500 und insbesondere von 1:50 bis 1:300 ein.

Die Hydroformylierungsreaktion in Gegenwart von Komplexliganden bezeichnet man häufig auch als modifizierte Variante, die üblicherweise bei Temperaturen von 50 bis 180°C, vorzugsweise von 100 bis 160°C und Gesamtdrücken von 0,2 bis 30 MPa, vorzugsweise von 1 bis 20 MPa durchgeführt wird.

Die Hydroformylierungsreaktion kann ebenfalls in Abwesenheit von Komplexliganden nach der unmodifizierten Variante durchgeführt werden. Solche, beispielsweise nicht mit Phosphinen oder Phosphiten modifizierte Übergangsmetallkatalysatoren und ihre Eignung als Katalysator zur Hydroformylierung sind aus der Literatur her bekannt und sie werden als unmodifizierte Übergangsmetallkatalysatoren bezeichnet. Es wird in der Fachliteratur angenommen, dass die Übergangsmetallverbindung HM(CO)₄ die katalytisch aktive Übergangsmetallspezies bei der unmodifizierten Übergangsmetallkatalyse ist, obgleich dies aufgrund der vielen in der Reaktionszone nebeneinander ablaufenden Chemismen nicht eindeutig bewiesen ist.

Vorzugsweise verwendet man als Übergangsmetalle der Gruppe VIII des Periodensystems der Elemente Kobalt, Rhodium, Iridium, Nickel, Palladium, Platin, Eisen oder Ruthenium und insbesondere Kobalt oder Rhodium. Der modifizierte oder unmodifizierte ÜbergangsmetaHkatalysator bildet sich unter den Bedingungen der Hydroformylierungsreaktion aus den eingesetzten Übergangsmetallverbindungen, wie deren Salzen, wie Chloriden, Nitraten, Sulfaten, Acetaten, Pentanoaten, 2-Ethylhexanoaten oder Isononanoaten, deren Chalkogeniden, wie Oxiden oder Sulfiden, deren Carbonylverbindungen, wie M₂(CO)₈, M₄(CO)₁₂, M₆(CO)₁₆, M₂(CO)₉, M₃(CO)₁₂, deren Organoübergangsmetallverbindungen, wie Carbonylacetylacetonaten oder Cyclooctadienylacetaten oder -chloriden, in Gegenwart von Kohlenmonoxid/ Wasserstoffgemischen. Dabei kann die Übergangsmetallverbindung als Feststoff oder zweckmäßigerweise in Lösung eingesetzt werden. Als Übergangsmetallverbindung, die als Katalysatorvorstufe verwendet wird, eignet sich insbesondere Rhodiumisononanoat, Rhodiumacetat, Rhodium-2-ethylhexanoat oder Kobaltisononanoat, Kobaltacetat oder Kobalt-2-ethylhexanoat, oder Co₂(CO)₈, Co₄(CO)₁₂, Rh₂(CO)₈, Rh₄(CO)₁₂ oder Rh₆(CO)₁₆ oder Cyclopentadienylrhodiumverbindungen, Rhodiumacetylacetonat oder Rhodiumdicarbonylacetylacetonat. Bevorzugt werden Rhodiumoxid und insbesondere Rhodiumacetat, Rhodium-2-ethylhexanoat und Rhodiumisononanoat eingesetzt.

Es ist aber auch möglich, den Übergangsmetallkatalysator in einer Vorcarbonylierungsstufe zunächst zu präformieren und ihn anschließend der eigentlichen Hydroformylierungsstufe zuzuführen. Die Bedingungen der Präformierung entsprechen dabei im Allgemeinen den Hydroformylierungsbedingungen.

Da im Allgemeinen die Verwendung von nicht mit Komplexliganden modifizierten Übergangsmetallkatalysatoren einen geringeren Übergangsmetallgehalt erfordert, arbeitet man bei der unmodifizierten Variante im Allgemeinen mit einer Übergangsmetallmenge von 1 bis 100 ppm, vorzugsweise 2 bis 30 ppm, bezogen auf das eingesetzte Octen. Ganz besonders wird Rhodium oder Kobalt in einer Menge von 2 bis 30 ppm, vorzugsweise von 5 bis 10 ppm, jeweils bezogen auf das eingesetzte Octen, verwendet.

Bei der Umsetzung des Octens mit Wasserstoff und Kohlenmonoxid zu Isononanal nach der unmodifizierten Variante arbeitet man zweckmäßigerweise bei höheren Drücken im Bereich von 5 bis 70 MPa, vorzugsweise von 5 bis 60 MPa und insbesondere von 10 bis 30 MPa. Geeignete Reaktionstemperaturen bewegen sich im Bereich von 50 bis 180°C, bevorzugt von 50 bis 150°C und insbesondere von 100 bis 150°C.

Die Zusammensetzung des Synthesegases, d. h. die Anteile von Kohlenmonoxid und Wasserstoff im Gasgemisch, kann in weiten Grenzen variiert werden. Im Allgemeinen setzt man Gemische ein, in denen das Molverhältnis von Kohlenmonoxid zu Wasserstoff 5 : 1 bis 1 : 5 beträgt. Üblicherweise ist dieses Verhältnis 1 : 1 oder weicht von diesem Wert nur wenig ab. Die olefinische Verbindung kann als solche oder in Lösung der Reaktionszone zugeführt werden. Geeignete Lösungsmittel sind Ketone wie Aceton, Methylethylketon, Acetophenon, niedrigere aliphatische Nitrile wie Acetonitril, Propionitril oder Benzonitril, Dimethylformamid, lineare oder verzweigte gesättigte aliphatische Monohydroxyverbindungen wie Methanol, Ethanol, Propanol und Isopropanol, aromatische Kohlenwasserstoffe wie Benzol oder Toluol und gesättigte cycloaliphatische Kohlenwasserstoffe wie Cyclopentan oder Cyclohexan.

Die Hydroformylierungsstufe kann sowohl diskontinuierlich als auch kontinuierlich durchgeführt werden. Die Gewinnung der gewünschten Aldehyde aus dem rohen Hydroformylierungsprodukt erfolgt nach konventionellen Verfahren, beispielsweise durch Destillation. Isononanal und weitere flüchtige Komponenten werden als Kopfprodukte abgezogen und bei Bedarf einer weiteren Feinreinigung unterzogen.

Die eingesetzten Übergangsmetallmengen fallen im Destillationsrückstand an und werden gegebenenfalls nach Zusatz von frischer Übergangsmetallverbindung und Entnahme eines Teils der im Verlauf der Reaktion gebildeten Aldehydkondensationsprodukte in die Reaktionszone zurückgeführt.

Das erhaltene Gemisch isomerer Isononanale wird aufgereinigt, zweckmäßigerweise durch Destillation, und anschließend durch Oxidation in die entsprechende Isononansäure überführt, vorzugsweise durch Oxidation in der Flüssigphase, obwohl andere Verfahrensausgestaltungen wie die Oxidation in der Gasphase nicht ausgeschlossen sind. Als Oxidationsmittel eignen sich übliche, zur Oxidation von aliphatischen Aldehyden geeignete Verbindungen wie Sauerstoff, sauerstoffenthaltende Gasgemische, Ozon, ozonhaltige Gasgemische, Peroxide, Persäuren, Metallsalze von Persäuren oder Übergangsmetalle in hohen Oxidationsstufen, beispielsweise Kaliumpermanganat oder Braunstein. Aufgrund der guten Verfügbarkeit verwendet man als Oxidationsmittel zweckmäßig molekularen Sauerstoff oder Gasgemische, die molekularen Sauerstoff enthalten. Weitere Bestandteile derartiger Gasgemische sind inerte Gase, z. B. Stickstoff, Edelgase und Kohlendioxid. Der Anteil der inerten Bestandteile des sauerstoffenthaltenden Gasgemisches beträgt bis zu 90 Vol%, insbesondere 30 bis 80 Vol%. Die bevorzugten Oxidationsmittel sind Sauerstoff oder Luft.

Die Oxidation kann entweder unter Katalysatorzusatz oder in Abwesenheit von Katalysatoren durchgeführt werden. Als Katalysatoren eignen sich Übergangsmetalle oder Verbindungen von Übergangsmetallen, die in geringen Mengen wie beispielsweise von 0,1 bis 5 ppm, berechnet als Übergangsmetall und bezogen auf eingesetzten Aldehyd, zugesetzt werden können wie Titan, Vanadium, Chrom, Molybdän, Mangan, Eisen, Kobalt, Nickel, Ruthenium, Rhodium, Palladium oder Kupfer. Eine solche Verfahrensführung wird beispielsweise in DE 100 10 771 C1 oder DE 26 04 545 A1 beschrieben.

Ebenfalls kann die Umsetzung in Anwesenheit von Alkali- oder Erdalkalimetallsalzen schwacher Säuren durchgeführt werden. Insbesondere bei der Oxidation von α-verzweigten Aldehyden, bei denen das dem Carbonylkohlenstoffatom benachbarte Kohlenstoffatom die Verzweigung trägt, empfiehlt der Stand der Technik die Anwesenheit von geringen Mengen an Alkalimetallcarboxylaten zur Selektivitätsverbesserung (DE 950 007, DE 100 10 771 C1). Auch kann eine Kombination von Alkali- oder Erdalkalimetallcarboxylaten mit Übergangsmetallverbindungen, wie in EP 1 854 778 A1 behandelt, verwendet werden.

Bei der Oxidation des Isononanals, das nach dem erfindungsgemäßen Verfahren ausgehend von 2-Ethylhexanol über die Dehydratisierung und Hydroformylierung des entsprechenden Octens hergestellt wird, ist die Anwesenheit von Alkali- oder Erdalkalimetallcarboxylaten empfehlenswert, im Allgemeinen in einer Menge von 1 bis 30 mmol, vorzugsweise von 1 bis 15 mmol und insbesondere von 1 bis 8 mmol, je Mol Aldehyd, berechnet als Alkali- oder Erdalkalimetall.

Es ist nicht erforderlich, die Alkali- oder Erdalkalimetallcarboxylate als einheitliche Verbindung einzusetzen. Es ist ebenfalls möglich, Gemische dieser Verbindungen zu verwenden, wobei man jedoch zweckmäßigerweise Isononanoate verwendet. Vorzugsweise setzt man jedoch einheitliche Verbindungen ein, beispielsweise Lithium-, Kalium-, Natrium-, Calcium- oder Bariumisononanoat.

Im Allgemeinen stellt man eine Alkali- oder Erdalkalimetallisononanoate enthaltende Lösung durch Neutralisation einer wässrigen, die Alkali- oder Erdalkalimetallverbindung enthaltenden Lösung mit einem Überschuss an Isononansäure her und setzt diese Lösung dem zu oxidierenden Isononanal zu. Als Alkali- oder Erdalkalimetallverbindungen eignen sich besonders die Hydroxide, Carbonate oder Hydrogencarbonate.

Es ist aber auch möglich, die Alkali- oder Erdalkalimetallisononanoate im Reaktionsgemisch zu erzeugen, indem man Alkali- oder Erdalkalimetallverbindungen zusetzt, die unter den Reaktionsbedingungen in die Isononanoate überführt werden. Beispielsweise lassen sich Alkali- oder Erdalkalimetallhydroxide, -carbonate, -hydrogencarbonate oder -oxide in der Oxidationsstufe einsetzen. Ihr Zusatz kann entweder in fester Form oder als wässrige Lösung erfolgen.

Die Umsetzung mit dem Oxidationsmittel, vorzugsweise mit Sauerstoff oder sauerstoffenthaltenden Gasen, wird in einem Temperaturbereich von 20 bis 100°C durchgeführt. Vorzugsweise arbeitet man zwischen 20 und 80°C, insbesondere zwischen 40 und 80°C. Die Temperaturführung, konstante oder variable Temperatur, kann den individuellen Erfordernissen des Ausgangsmaterials und den Reaktionsgegebenheiten angepasst werden.

Die Umsetzung der Reaktionspartner erfolgt bevorzugt bei Atmosphärendruck. Die Anwendung erhöhten Drucks ist jedoch nicht ausgeschlossen.

Üblicherweise arbeitet man in einem Bereich von Atmosphärendruck bis 1,5 MPa, vorzugsweise bei Atmosphärendruck bis 0,8 MPa.

Die zur Umwandlung des Isononanals in die entsprechende Isononansäure erforderliche Reaktionszeit hängt unter anderem ab von der Reaktionstemperatur und dem Mengenverhältnis der Reaktanten zueinander. Normalerweise beträgt sie 30 Minuten bis 20 Stunden, insbesondere 2 bis 8 Stunden.

Isononanal kann als solches oder gelöst in einem, unter den Reaktionsbedingungen inerten, Lösungsmittel eingesetzt werden. Beispiele für geeignete Lösungsmittel sind Ketone wie Aceton, Ester, z. B. Ethylacetat, Kohlenwasserstoffe, z. B. Toluol und Nitrokohlenwasserstoffe wie Nitrobenzol. Die Konzentration des Aldehyds wird durch seine Löslichkeit in dem Lösungsmittel begrenzt.

Der Oxidationsschritt kann absatzweise oder kontinuierlich durchgeführt werden. Eine Rückführung nicht umgesetzter Reaktionsteilnehmer ist in beiden Fällen möglich.

Bei der erhaltenen Isononansäure ausgehend von 2-Ethylhexanol handelt es sich um ein Gemisch stellungsisomerer aliphatischer C9-Monocarbonsäuren mit in α-Position unverzweigten und einfachverzweigten Isononansäuren als Hauptkomponenten.

Nach der gaschromatographischen Analyse gemäß DIN 51405 (FI.-%) liegen als Hauptkomponenten 4-Methyloctansäure, 6-Methyloctansäure, 2,5-Dimethylheptansäure, 2,3-Dimethylheptansäure, 3-Ethylheptansäure, 2-Ethylheptansäure und 2-Ethyl-4-methylhexansäure vor sowie geringe Mengen an 2-Propyl-3-methylpentansäure und 2-Methyloctansäure. Geringe Mengen an n-Nonansäure sind ebenfalls zugegen.

Die nach dem erfindungsgemäßen Verfahren hergestellte Isononansäure ist dadurch charakterisiert, dass die Hauptkomponenten 4-Methyloctansäure, 6-Methyloctansäure, 2,5-Dimethylheptansäure, 2,3-Dimethylheptansäure, 3-Ethylheptansäure, 2-Ethylheptansäure und 2-Ethyl-4-methylhexansäure in Summe mindestens 80 mol%, bezogen auf den Gesamtgehalt an stellungsisomeren aliphatischen C9-Monocarbonsäuren, ausmachen.

Aus dem nach der Oxidation anfallenden Rohsäuregemisch wird mittels Destillation unter üblichen Bedingungen die reine Isononansäure gewonnen. Der die Alkali- oder Erdalkalimetallisononanoate und gegebenenfalls Übergangsmetalle enthaltende Destillationsrückstand wird abgetrennt und kann dem Einsatzaldehyd, gegebenenfalls nach Zugabe von frischen Alkali- oder Erdalkalimetallisononanoaten oder Alkali- oder Erdalkalimetallverbindungen, die unter den Reaktionsbedingungen in die Isononanoate übergehen, sowie gegebenenfalls von frischen Übergangsmetallverbindungen wieder zugeführt werden.

Nach einer bewährten Ausführungsform des erfindungsgemäßen Verfahrens legt man Isononanal in einem geeigneten Reaktor, z. B. in einem mit einem Anströmboden versehenen Rohrreaktor, der gegebenenfalls noch Füllkörper enthält, vor und leitet den Sauerstoff oder das sauerstoffenthaltende Gasgemisch von unten durch den Aldehyd.

Entsprechend einer weiteren Ausführungsform verwendet man als Reaktor einen Rieselturm, der Füllkörper enthält. Über die Füllung lässt man den Aldehyd herabrieseln und leitet in den Turm gleichzeitig im Gleich- oder Gegenstrom Sauerstoff oder ein sauerstoffenthaltendes Gasgemisch ein.

Die nach dem erfindungsgemäßen Verfahren hergestellte Isononansäure wird anschließend mit Alkoholen zu Carbonsäureestern verestert.

Die unmittelbare Veresterung von Alkoholen mit Carbonsäuren gehört zu den Grundoperationen der organischen Chemie und wird beispielsweise in DE 10 2009 048 771 A1, DE 10 2009 048 772 A2 oder DE 199 40 991 A behandelt.

Um die Reaktionsgeschwindigkeit zu erhöhen, verwendet man einen der Reaktanten im Überschuss und trennt das im Verlauf der Reaktion gebildete Wasser ab, um das Gleichgewicht entsprechend dem Massenwirkungsgesetz zur Seite des Reaktionsproduktes, also des Carbonsäureesters, zu verschieben. Je nach Lage der Siedepunkte der Reaktionskomponenten setzt man die Isononansäure oder den Alkohol im Überschuss ein. Üblicherweise wird die leichter flüchtige Komponente im Überschuss eingesetzt, die zusammen mit dem Reaktionswasser aus dem Reaktionsgefäß entfernt und in einen nachgeschalteten Phasentrenner geleitet wird, in dem sich die organische Phase von der wässrigen trennt, die aus dem Prozess ausgeschleust wird. Gegebenenfalls bildet die leichter flüchtige Komponente mit Wasser unter den Reaktionsbedingungen auch ein Azeotrop und vermag als Schleppmittel das Reaktionswasser zu entfernen. Anwendung findet auch die Azeotropdestillation in Gegenwart eines mit Wasser nicht mischbaren Lösemittels wie Hexen-1, Cyclohexan oder Toluol, das Erhitzen des Reaktionsgemisches unter Durchleiten eines inerten Gases, die Umsetzung der Ausgangsstoffe Alkohol und Isononansäure bei vermindertem Druck oder in Gegenwart eines Trocknungsmittels, um das Reaktionswasser zu entfernen. Es ist empfehlenswert, das Reaktionsgefäß mit einer Fraktionierkolonne auszustatten, die 2 bis 10 theoretische Böden aufinreist, um das aus dem Reaktionsgefäß abgeführte Gemisch aus Wasser und flüchtiger Reaktionskomponente in eine wasserabgereicherte Fraktion, die in das Reaktionsgefäß zurückfließt, und in eine wasserangereicherte Fraktion aufzuspalten, die als Kopffraktion abgeführt wird.

Die Veresterung der Isononansäure mit Alkoholen kann ohne Einsatz eines Katalysators durchgeführt werden. Diese Variante der Umsetzung hat den Vorteil, dass man vermeidet, dem Reaktionsgemisch Fremdstoffe zuzuführen, die zu einer unerwünschten Verunreinigung des Carbonsäureesters führen kann. Allerdings muss man dann im Allgemeinen höhere Reaktionstemperaturen einhalten, weil nur so gewährleistet ist, dass die Umsetzung mit ausreichender, d. h. wirtschaftlich vertretbarer Geschwindigkeit abläuft.

Zu beachten ist in diesem Zusammenhang, dass die Steigerung der Temperatur zu einer thermischen Schädigung des Carbonsäureesters führen kann. Daher lässt sich nicht immer die Verwendung eines Katalysators, der die Umsetzung erleichtert und die Reaktionsgeschwindigkeit erhöht, vermeiden. Häufig kann der Katalysator ein Überschuss der Isononansäure sein, die gleichzeitig Reaktionskomponente des Alkohols ist, so dass die Reaktion autokatalytische abläuft. Im Übrigen sind die üblichen Veresterungskatalysatoren zur Beeinflussung der Reaktionsgeschwindigkeit geeignet wie Schwefelsäure, Ameisensäure, Polyphosphorsäure, Methansulfonsäure oder p-Toluolsulfonsäure und ebenso Kombinationen derartiger Säuren. Ebenfalls verwendet werden können metallhaltige Katalysatoren wie Titan, Zirkonium oder Zinn enthaltende Katalysatoren, beispielsweise die entsprechenden Alkoholate oder Carboxylate. Auch unter Reaktionsbedingungen feste, im Reaktionssystem unlösliche, katalytisch wirksame Verbindungen wie Alkalioder Erdalkalimetallhydrogensulfate, beispielsweise Natriumhydrogensulfat können eingesetzt werden. Feste Katalysatoren werden nach Beendigung der Veresterung durch einfache Filtration aus dem Reaktionsgemisch entfernt. Die Menge des eingesetzten Katalysators kann sich über einen weiten Bereich erstrecken. Es können zwischen 0,001 Gew.-% und 5 Gew.-% Katalysator, bezogen auf das Reaktionsgemisch, verwendet werden. Da größere Katalysatormengen jedoch kaum Vorteile ergeben, beträgt die Katalysatorkonzentration üblicherweise 0,001 bis 1,0, vorzugsweise 0,01 bis 0,5 Gew.-% jeweils bezogen auf das Reaktionsgemisch.

Es ist ebenfalls möglich, die Veresterungsreaktion in Gegenwart eines Adsorbens durchzuführen. Man verwendet dabei poröse, großflächige feste Materialien, die üblicherweise in der chemischen Praxis sowohl im Labor als auch in technischen Anlagen eingesetzt werden. Beispiele für solche Materialien sind oberflächenreiche Polykieselsäuren wie Silicagele (Kiesel-Xerogele), Kieselgel, Kieselguhr, oberflächenreiche Aluminiumoxide und Aluminiumoxidhydrate, mineralische Materialien, wie Tone, Carbonate oder Aktivkohle. Besonders bewährt hat sich Aktivkohle. Im Allgemeinen ist das Adsorbens feinteilig in der Reaktionslösung suspendiert, die durch intensives Rühren oder durch Einleiten eines Inertgases bewegt wird. Dadurch wird ein inniger Kontakt zwischen der flüssigen Phase und dem Adsorbens erreicht. Das Massenverhältnis von flüssiger Phase zu Adsorbens kann weitgehend frei und somit den individuellen Erfordernissen entsprechend eingestellt werden. Es hat sich bewährt, je 100 Gew.-Teile flüssiger Phase 0,05 bis 30, vorzugsweise 0,1 bis 5 und insbesondere 0,1 bis 1 Gew.-Teile Adsorbens einzusetzen. Nach beendeter Umsetzung kann das Adsorbens aus dem Prozess abgetrennt und in das Veresterungsgefäß zurückgeführt und wieder eingesetzt werden. Der Wiedereinsatz ist sooft möglich, bis die Entfärbekraft des Adsorbens erschöpft ist. Es ist aber auch möglich, das Adsorbens in dem Rohprodukt zu belassen und während des Aufarbeitungsverfahrens an einer beliebigen aber zweckmäßigen Stufe abzutrennen.

Das nach beendeter Umsetzung erhaltene Reaktionsgemisch enthält neben dem Carbonsäureester als dem gewünschten Reaktionsprodukt gegebenenfalls nicht umgesetzte Einsatzstoffe, insbesondere den im Überschuss eingesetzten Reaktionspartner, und wird nach bekannten Verfahren aufgearbeitet.

Nach Abtrennung nicht umgesetzter und überschüssiger Einsatzprodukte wird der erhaltene Rohester einer Behandlung mit Wasserdampf unterzogen, die beispielsweise in einfacher Form durch Einleiten von Wasserdampf in das Rohprodukt erfolgen kann. Ein Vorteil der Wasserdampfbehandlung ist, dass in ihrem Verlauf noch vorhandener Katalysator zerstört und in gut abfiltrierbare Hydrolyseprodukte überführt wird. Wird die Veresterungsreaktion in Gegenwart eines Adsorbens durchgeführt, erleichtert das bereits anwesende Adsorbens die Abscheidung der Katalysatorfolgeprodukte. Ansonsten kann es sich als vorteilhaft erweisen, ein Adsorbens zu Beginn der Wasserdampfbehandlung zuzusetzen. Die Anwesenheit eines Adsorbens während der Wasserdampfbehandlung wirkt sich ebenfalls vorteilhaft auf die Farbe und auf die Farbstabilität des Carbonsäureesters aus. Es ist aber auch möglich, nach beendeter Veresterungsreaktion und Abtrennung überschüssiger Ausgangsverbindungen, also vor Durchführung der Wasserdampfdestillation, das Adsorbens abzufiltrieren.

An die Wasserdampfbehandlung schließt sich gegebenenfalls nach Filtration des Adsorbens und weiterer angefallener Feststoffe die Trocknung des Carbonsäureesters an, beispielsweise in dem man ein inertes Gas durch das Produkt bei erhöhter Temperatur leitet. Es kann auch bei erhöhter Temperatur gleichzeitig ein Unterdruck angelegt werden und gegebenenfalls ein inertes Gas durch das Produkt geleitet werden. Auch kann trockener Wasserdampf bei Unterdruck durch den Carbonsäureester geblasen werden. Auch ohne Einwirken eines inerten Gases kann nur bei erhöhter Temperatur oder nur bei geringerem Druck gearbeitet werden. Darauf wird der Rohester, falls noch nicht erfolgt, filtriert, um ihn von den Feststoffen, den Hydrolyseprodukten des Katalysators und dem Adsorbens, falls in der Veresterungsstufe oder vor der Wasserdampfbehandlung zugesetzt, zu befreien. Im Allgemeinen fällt der gewünschte Carbonsäureester während der Trocknungsstufe als Rückstand an. In besonderen Fällen kann sich noch eine fraktionierte Destillation im Vakuum anschließen.

Unabhängig davon, ob in der Veresterungsstufe bereits ein Adsorbens zugegen war und bereits zu Beginn der Aufarbeitungsmaßnahmen abfiltriert wurde oder erst nach der Trocknung, kann es sich als zweckmäßig erweisen, den Carbonsäureester einer nochmaligen Nachbehandlung mit einem Absorbens zu unterziehen oder mit einem Oxidationsmittel zu behandeln, beispielsweise mit einer wässrigen Peroxidlösung oder mit Ozon oder ozonhaltigen Gasen, um die Farbzahl zu verbessern.

An geeigneten Stellen des Aufarbeitungsverfahrens kann der Rohester ebenfalls mit einem alkalischen Reagenz, beispielsweise mit einer wässrigen Natriumhydroxid- oder Natriumcarbonatlösung behandelt werden, so zum Beispiel nach Abtrennung der überschüssigen Ausgangsverbindungen und vor der Maßnahme der Wasserdampfdestillation oder während der Wasserdampfdestillation. Auch kann nach Abtrennung der überschüssigen Ausgangsverbindungen eine Teilmenge an alkalischem Reagenz zugegeben werden und die Restmenge im Verlauf der Wasserdampfdestillation. Auf diese Weise kann die Säurezahl des Carbonsäureesters gemäß den Spezifikationsanforderungen eingestellt werden.

Die als Ausgangsstoffe für die Veresterungsstufe eingesetzten Alkohole sind ein- oder mehrwertige Alkohole, die im Allgemeinen der allgemeinen Formel (I) genügen

R(OH)ₙ (I)

in der R einen aliphatischen, cycloaliphatischen oder aromatischen Kohlenwasserstoffrest mit 1 bis 20, vorzugsweise 2 bis 10 Kohlenstoffatomen und n eine ganze Zahl von 1 bis 8, vorzugsweise 1, 2, 3, 4, 5 oder 6 ist.

Geeignete Alkohole sind beispielsweise die einwertigen Alkohole Propanol, iso-Butanol, n-Butanol, n-Hexanol, n-Heptanol, 2-Ethylhexanol, n-Octanol, 3,5,5-Trimethylhexanol, Isononanol, n-Nonanol oder Benzylalkohol, die zweiwertigen Alkohole Ethylenglykol, 1,2-Propandiol, 1,3-Propandiol, 1,3-Butandiol, 2,3-Butandiol, 1,4-Butandiol, Neopentylglykol, 2,2-Dimethylolbutan, 2,2,4-Trimethylpentan-1,3-diol, 1,5-Pentandiol, 1,2-Hexandiol, 1,6-Hexandiol, Ethylenglykol oder 3(4),8(9)-Bishydroxymethyl-tricyclo[5.2.1.0^{2.6}]decan, die dreiwertigen Alkohole Trimethylolethan, Trimethylolpropan, Trimethylolbutan, Glycerin sowie der vierwertige Alkohol Pentaerythrit.

Ebenfalls kann die erfindungsgemäß hergestellte Isononansäure mit Glycidylalkohol zum Glycidylester umgesetzt werden.

Als Alkohole eignen sich ebenfalls mehrwertige Alkohole oder Polyole mit einer Ethergruppe der allgemeinen Formel

H-(-O-[-CR¹R²-]ₘ-)ₒ-OH (II)

in der R¹ und R² unabhängig voneinander Wasserstoff, einen Alkylrest mit 1 bis 5 Kohlenstoffatomen, vorzugsweise Methyl, Ethyl oder Propyl, oder einen Hydroxyalkylrest mit 1 bis 5 Kohlenstoffatomen, vorzugsweise der Hydroxymethylrest, m eine ganze Zahl von 1 bis 10, vorzugsweise 1 bis 8 und insbesondere 1, 2, 3 oder 4, o eine ganze Zahl von 2 bis 15, vorzugsweise 2 bis 8 und insbesondere 2, 3, 4 oder 5 bedeuten.

Geeignete mehrwertige Alkohole der allgemeinen Formel (II), die eine Ethergruppe enthalten, sind beispielsweise Di-Trimethylolpropan, Di-Pentaerythrit, Diethylenglykol, Triethylenglykol, Tetraethylenglykol, Dipropylenglykol, Tripropylenglykol oder Tetrapropylenglykol.

In Abhängigkeit von den Siedepunkten der eingesetzten Alkohole in Bezug auf den Siedepunkt der Isononansäure setzt man die schwerer flüchtige Reaktionskomponente im Unterschuss ein und in Bezug hierzu wird die leichter flüchtige Reaktionskomponente in einem 10 bis 50%-igen molaren, vorzugsweise in einem 20 bis 40%-eigen molaren Überschuss je mol zu verestemder funktioneller Gruppe eingesetzt. Bei der Veresterung hochsiedender mehrwertiger Alkohole wie Neopentylglykol, Trimethylolpropan, Di-Trimethylolpropan, Pentaerythrit, Di-Pentaerythrit, Triethylenglykol oder Tetraethylenglykol wird zweckmäßigerweise Isononansäure im Überschuss eingesetzt, die auf einfache Weise destillativ aus dem Rohester abgetrennt werden kann. Werden hingegen niedrig siedende Alkohole wie n-Propanol oder iso-Butanol mit der Isononansäure verestert, wird der entsprechende Alkohol im Überschuss zur Reaktion gebracht.

Die Veresterungsreaktion kann sowohl absatzweise als auch kontinuierlich durchgeführt werden.

Die erfindungsgemäßen Ester eignen sich hervorragend als Weichmacher für alle gängigen hochpolymeren thermoplastischen Kunststoffe. Ebenso können sie als Schmiermittel hervorragend verwendet werden. Zur Herstellung von Schmiermitteln wird häufig Isononansäure in Mischung mit anderen Monocarbonsäuren verestert. Beispielsweise wird eine Mischung enthaltend Isononansäure, n-Pentansäure und n-Heptansäure mit mehrwertigen Alkoholen umgesetzt.

In den folgenden Beispielen wird die Herstellung von Isononansäure ausgehend von 2-Ethylhexanol und die Herstellung von Carbonsäureestern beschrieben.

### Beispiele

### I. Dehydratisierung von 2-Ethylhexanol

Zur Dehydratisierung wurde ein Quarzrohr mit einer Länge von 1,3 Meter und einem Durchmesser von 0,03 Meter verwendet, bei dem sich die beheizte Zone über 1,1 Meter erstreckte. Das Quarzrohr wurde mit 250 ml des sauren Katalysators Al 3996 der Firma BASF SE in Form von 3x3 Millimeter großen Tabletten bestückt. Das Totvolumen wurde mit Glasringen aufgefüllt. 2-Ethylhexanol wurde in einem vorgeschalteten Verdampfer verdampft und mit Hilfe eines Stickstoffstromes bei Normaldruck über die Katalysatorschüttung bei einer Temperatur von 350°C und mit einer Belastung von 0,5 Liter je Liter Katalysatorvolumen und Stunde gefahren. Das erhaltene Reaktionsgemisch wurde in einem nachgeschalteten Auffanggefäß kondensiert und die wässrige Phase wurde abgetrennt. Die angefallene organische Phase wies folgende gaschromatographisch ermittelte Zusammensetzung auf (Fl.-%, gemäß DIN 51405):

| | |
|---|---|
| Vorlauf/C4-C7-Kohlenwasserstoffe | 0,3 |
| andere C8-Olefine | 9,6 |
| 2-Ethyl-1-hexen | 7,6 |
| cis-3-Methyl-3-hepten | 14,6 |
| trans-3-Methyl-3-hepten | 28,8 |
| cis-3-Methyl-2-hepten | 16,2 |
| trans-3-Methyl-2-hepten | 23,9 |
| n-Octene | 0,8 |
| Nachlauf | 0,1 |

### II. Hydroformylierung des gemäß Schritt I. erhaltenen Octens

Das nach Schritt 1 erhaltene rohe Octen wurde in Gegenwart von 5 ppm Rhodium, zugegeben in Form einer Lösung von Rhodium-2-ethylhexanoat in 2-Ethylhexanol und bezogen auf Octeneinsatz, bei einer Temperatur von 140°C und bei einem Synthesegasdruck von 19 MPa über einen Zeitraum von drei Stunden hydroformyliert. Die molare Zusammensetzung des Synthesegases betrug 1 Mol Wasserstoff zu 1 Mol Kohlenmonoxid. Das erhaltene rohe Hydroformylierungsprodukt wies folgende gaschromatographisch ermittelte Zusammensetzung (FI.-%, gemäß DIN 51405) auf:

| | |
|---|---|
| Vorlauf | 0,1 |
| C8-Kohlenwasserstoffe | 8,5 |
| Zwischenlauf | 0,2 |
| Isononanal | 88,1 |
| n-Nonanal | 1,4 |
| Nachlauf | 1,7 |

Die Ergebnisse weiterer Hydroformylierungsversuche mit einem über die Dehydratisierung von 2-Ethylhexanol erhaltenen Octen sind in der nachfolgenden Tabelle 1 zusammengestellt. Vor Einsatz wurde das rohe Octen an einer Claisenbrücke zur Nachlaufabtrennung bei einer Kopftemperatur von 119-122°C und bei Normaldruck destilliert. Die Einsatzoctene sowie die erhaltenen Reaktionsprodukte wurden gaschromatographisch analysiert (Angabe in FI.-%, gemäß DIN 51405).

**Tabelle 1: Hydroformylierung von Octenen, erhalten durch die 2-Ethylhexanoldehydratisierung**

| **Beispiel** | **IIa** | **IIb** |
|---|---|---|
| Einsatz Edukt | destilliert | destilliert |

| **GC-Analyse Edukt (%)** | | |
|---|---|---|
| Vorlauf/C4-C7-Kohlenwasserstoffe | 0,3 | 0,4 |
| andere C8-Olefine | 5,9 | 7,7 |
| 2-Ethyl-1-hexen | 9,3 | 9,2 |
| cis-3-Methyl-3-hepten | 15,2 | 15,0 |
| trans-3-Methyl-3-hepten | 27,4 | 27,1 |
| cis-3-Methyl-2-hepten | 16,1 | 15,6 |
| trans-3-Methyl-2-hepten | 25,2 | 24,7 |
| n-Octene | 0,5 | 0,2 |
| Nachlauf | 0,1 | 0,1 |

| **Versuchsbedingungen** | | |
|---|---|---|
| Rh-Konzentration [ppm], bezogen auf Octeneinsatz | 20 | 10 |
| Druck [MPa] | 19 | 27 |
| Temperatur [°C] | 140 | 140 |
| Reaktionszeit [h] | 2 | 2 |

| **GC-Analyse Produkt (%)** | | |
|---|---|---|
| Vorlauf | 0,1 | 0,1 |
| C8-Kohlenwasserstoffe | 2,5 | 1,1 |
| Zwischenlauf | 0,3 | 0,1 |
| iso-Nonanale | 90,8 | 94,7 |
| n-Nonanal | 2,0 | 1,4 |
| Nachlauf | 4,3 | 2,6 |

Die unter Verwendung von Triphenylphosphin als Komplexligand durchgeführten Hydroformylierungsversuche mit dem über die Dehydratisierung von 2-Ethylhexanol erhaltenen Octen sind in der nachfolgenden Tabelle 2 zusammengestellt. Es kam undestillierte Ware zum Einsatz. Die Einsatzoctene sowie die erhaltenen Reaktionsprodukte wurden gaschromatographisch analysiert (Angaben in FI.-%, gemäß DIN 51405).

**Tabelle 2: Hydroformylierung von Octenen, erhalten durch die 2-Ethylhexanoldehydratisierung, Zusatz von Triphenylphosphin**

| **Beispiel** | **IIc** | **IId** | **IIe** | **IIf** |
|---|---|---|---|---|
| Einsatz Edukt | undestilliert, roh | undestilliert, roh | undestilliert, roh | undestilliert, roh |

| **GC-Analyse Edukt (%)** | | | | |
|---|---|---|---|---|
| C4-C7 Kohlenwasserstoffe | 0,3 | 0,3 | 0,3 | 0,4 |
| Andere C8-Olefine | 19,1 | 19,1 | 19,1 | 11,6 |
| 2-Ethyl-1-hexen | 7,9 | 7,9 | 7,9 | 8,6 |
| 3-Methyl-3-hepten | 36,5 | 36,5 | 36,5 | 40,0 |
| 3-Methyl-2-hepten | 36,2 | 36,2 | 36,2 | 39,3 |
| Nachlauf | <0,01 | <0,01 | <0,01 | <0,1 |

| **Versuchsbedingungen** | | | | |
|---|---|---|---|---|
| Rh-Konzentration [ppm], bezogen auf Octeneinsatz | 10 | 10 | 10 | 10 |
| Äquivalente TPP | 3 | 50 | 100 | 3 |
| Druck [MPa] | 18 | 27 | 18 | 14 |
| Temperatur [°C] | 140 | 140 | 140 | 160 |
| Reaktionszeit [h] | 1 | 2 | 1 | 2 |

| **GC-Analyse Produkt (%)** | | | | |
|---|---|---|---|---|
| Vorlauf | 0,1 | 0,1 | 0,1 | 0,1 |
| C8 Kohlenwasserstoffe | 52,2 | 70,9 | 81,7 | 14,1 |
| Zwischenlauf | 0,8 | 0,1 | 0,1 | 1,9 |
| Iso-Nonanale | 45,7 | 28,3 | 17,6 | 76,1 |
| n-Nonanal | 0,5 | 0,1 | 0,1 | 0,5 |
| Nachlauf | 0,7 | 0,4 | 0,4 | 7,3 |

### III. Oxidation des nach Schritt II. erhaltenen Isononanals zu Isononansäure

Aus dem nach Beispiel IIa erhaltenen Isononanal wurden zunächst Leichtsieder und unumgesetztes Olefin als Kopfprodukt an einer 24 Bödenkolonne bei 200 hPa, einer Sumpftemperatur von 120°C und einem Rücklaufverhältnis von 2:1 abgetrennt. Nach Leichtsiederabtrennung wurde die Sumpftemperatur auf 140-150°C angezogen und das Isononanal über Kopf abgezogen (Siedepunkt bei 100 hPa: 110-113°C), während Hochsieder im Destillationssumpf verblieben.

Das erhaltene Isononanal wies folgende gaschromatographisch ermittelte Zusammensetzung sowie folgende Kennzahlen auf und wurde für die nachfolgende Flüssigphasenoxidation eingesetzt.

**Tabelle 3: Gaschromatographische Analyse (FI.-%, gemäß DIN 51405) des Isononanals ausgehend von 2-Ethylhexanol**

| | |
|---|---|
| Vorlauf/C8-Kohlenwasserstoffe | 0,2 |
| Zwischenlauf | 0,4 |
| 2-Ethyl-4-methylhexanal | 10,8 |
| 2-Propyl-3-methypentanal | 3,6 |
| 2,5-Dimethylheptanal | 21,9 |
| 2,3-Dimethylheptanal (Isomer) | 4,8 |
| 2,3-Dimethylheptanal (Isomer) + 2-Ethylheptanal | 8,4 |
| 2-Methyloctanal | 1,7 |
| 3-Ethylheptanal | 10,4 |
| 4-Methyloctanal | 20,6 |
| 4,5-Dimethylheptanal | 0,6 |
| 6-Methyloctanal | 11,0 |
| andere i-Nonanale | 1,8 |
| n-Nonanal | 0,9 |
| Nachlauf | 2,9 |

**Tabelle 4: Kennzahlen des Isononanals ausgehend von 2-Ethylhexanol**

| Kennzahl / Einheit | DIN/ASTM | Wert |
|---|---|---|
| V₂₀ (mm²/s) | ASTM D 445 | 1,536 |
| V₄₀ ( mm²/s) | | 1,179 |
| Erstarrungspunkt (°C) | | -100 |
| d^{20/4} (g/cm³) | DIN 51757, Verf. D/ASTM D 4052 | 0,827 |
| d^{50/4} (g/cm³) | | 0,811 |
| n^{20/D} | DIN 51423-2/ ASTM D 1747 | 1,424 |
| CO-Zahl (mg KOH/g) | DIN 53173 | 339/349 |
| Flammpunkt (°C) | DIN EN ISO 2719 | 60 |
| Platin/Kobalt-Farbzahl Hazen | DIN EN ISO 6271/ ASTM D 1209 | 15 |

Die Flüssigphasenoxidation des Isononanals zu Isononansäure erfolgte ohne Lösungsmittelzusatz in einem Blasensäulenreaktor bei 50°C mit reinem Sauerstoff bei Normaldruck über einen Zeitraum von 6 Stunden. Dem Einsatzaldehyd wurde eine 50 Gew.-%ige wässrige Lösung von Kaliumhydroxid in einer solchen Menge zugesetzt, dass pro Mol Isononanal 50 mmol Kalium vorlagen.

Die erhaltene Rohsäure wurde anschließend an einer 4,5 Bödenkolonne bei einer Sumpftemperatur von 148 bis 159°C und bei einer Kopftemperatur von 136-139°C bei 20 hPa destilliert. Leichtsieder und unumgesetzter Aldehyd wurden als Vorlauffraktion abgetrennt und Schwersieder verblieben im Destillationsrückstand. Die Destillationsausbeute an Isononansäure betrug 84,7% mit einer gaschromatographisch ermittelten Reinheit von 98,8%.

Die erhaltene Isononansäure wies folgende nach DIN 51405 gaschromatographisch ermittelte Zusammensetzung (FI.-%) auf:

**Tabelle 5: Gaschromatographische Analyse der Isononansäure ausgehend von 2-Ethylhexanol (FI.-%, gemäß DIN 51405)**

| | |
|---|---|
| Vorlauf | 0,4 |
| 2-Ethyl-4-methylhexansäure | 9,3 |
| 2-Propyl-3-methylpentansäure | 3,0 |
| 2,5-Dimethylheptansäure + 2,3-Dimethylheptansäure (Isomer) | 25,7 |
| 2,3-Dimethylheptansäure (Isomer) | 8,4 |
| 3-Ethylheptansäure + 2-Ethylheptansäure | 12,9 |
| 2-Methyloctansäure | 0,8 |
| 4-Methyloctansäure | 20,9 |
| 6-Methyloctansäure | 12,3 |
| n-Nonansäure | 0,3 |
| andere i-Nonansäuren | 5,2 |
| Nachlauf | 0,8 |

Die für die Isononansäure ermittelten Kennzahlen sind in der Tabelle 6 zusammengestellt.

**Tabelle 6: Kennzahlen der Isononansäure ausgehend von 2-Ethylhexanol**

| Kennzahl / Einheit | DIN/ASTM | Wert |
|---|---|---|
| V₂₀ (mm²/s) | ASTM D 445 | 10,68-11,18 |
| V₄₀ (mm²/s) | | 5,83-5,88 |
| V₅₀ (mm²/s) | | 4,50 |
| d^{20/4} (g/cm³) | DIN 51757, Verf. D/ ASTM D 4052 | 0,906-0,907 |
| d^{40/4} (g/cm³) | | 0,891 |
| d^{50/4} (g/cm³) | | 0,883-0,884 |
| n^{20/D} | DIN 51 423-2/ ASTM D 1747 | 1,432-1,433 |
| Erstarrungspunkt (°C) | | -81 |
| Siedepunkt (°C) bei 1013 hPa | DIN 53171 / ASTM D 1078 | 241-242 |
| Säurezahl mg KOH/g | DIN EN ISO 2114/ ASTM D 1613 | 351 |
| Flammpunkt (°C) | DIN EN ISO 2719 | 129 |
| Platin/Kobalt-Farbzahl Hazen | DIN EN ISO 6271/ ASTM D 1209 | 7 |

### IV. Veresterung der nach Schritt III. erhaltenen Isononansäure

Die Veresterung von Polyolen mit der gemäß Schritt III hergestellten Isononansäure wurde in einem beheizbaren 2 L - Vierhalskolben durchgeführt, der mit Rührer, Innenthermometer und einem Wasserabscheider ausgestattet wurde.

In dem Kolben wurden das Polyol und die Carbonsäure im 20 mol%igem Überschuss, bezogen auf jede zu veresternde Alkoholgruppe, und je nach Ansatz 0,05 mol% Tetra(isopropyl)orthotitanat, bezogen auf das Polyol, vorgelegt. Bei den Beispielen IV/2, IV/6 und IV/7 wurde ohne Katalysator gearbeitet. Die anwesende Carbonsäure wirkte autokatalytisch. Bei den Beispielen IV/1 bis IV/6 führte man die Veresterungsreaktion in Gegenwart von Aktivkohle durch. Bei den Beispielen IV/6 und IV/7 wurde eine Mischung aus Isononansäure, n-Pentansäure und n-Heptansäure eingesetzt. Die verwendeten Polyole, die Veresterungsbedingungen sowie die angewandten Bedingungen bei der Aufarbeitung des Rohesters sind in der nachfolgenden Tabelle 7 zusammengestellt.

Unter Rühren und unter Anlegen des angegebenen Unterdrucks wurde der Ansatz auf 200°C erhitzt und gebildetes Reaktionswasser an dem Wasserabscheider abgenommen. Nach einer Reaktionszeit von 2 Stunden bei dieser Stufe wurde der Druck auf 600 hPa erniedrigt und die Temperatur auf 220°C erhöht. Der Reaktionsverlauf wurde durch kontinuierliche Auswaage des über den Wasserabscheider ausgeschleusten Reaktionswassers sowie durch Probenahme und gaschromatographische Untersuchung der Proben verfolgt. Eine nochmalige Absenkung des Unterdrucks bis auf 300 hPa wurde abhängig vom Veresterungsgrad vorgenommen. Bei einem gaschromatographisch ermittelten Gehalt an Ester von mindestens 98,0% wurde die Reaktion durch Abkühlen des Ansatzes beendet. Die jeweilige Veresterungsdauer kann aus der Tabelle 7 entnommen werden.

Aus den nach den Beispielen IV/1 -IV/7 erhaltenen Reaktionsansätzen wurde zunächst die überschüssige und nicht umgesetzte Carbonsäure abgetrennt. Es folgte eine Wasserdampfdestillation und anschließende Trocknung. Nach Filtration der zugesetzten Aktivkohle bei Normaldruck und Raumtemperatur erhielt man als Rückstand einen hellfarbigen Polyolester mit der in Tabelle 7 angegebenen gaschromatographisch ermittelten Zusammensetzung (FI.-%, gemäß DIN 51405) und den gemessenen Kennzahlen.

**Tabelle 7: Veresterung der gemäß Schritt III hergestellten Isononansäure ausgehend von 2-Ethylhexanol mit Polyolen**

| **Veresterung** | | **1** | **2** | **3** | **4** | **5** | **6** | **7** |
|---|---|---|---|---|---|---|---|---|
| Polyol | | TEG | NPG | 1,3 BG | TCD | TMP | Pentaerythrit | Di-Pentaerythrit |
| Menge an Polyol | Mol | 0,67 | 0,66 | 1,00 | 1,0 | 1,00 | 0,45 | 0,29 |
| Iso-Nonansäure | Mol | 1,73 | 1,72 | 2,60 | 2,60 | 3,60 | 0,76 | 0,66 |
| n-Heptansäure | Mol | | | | | | 0,73 | 0,71 |
| n-Pentansäure | Mol | | | | | | 0,72 | 0,77 |
| Aktivkohle | g | 1,0 | 0,7 | 0,9 | 2,7 | 2,18 | 2,55 | - |
| Tetra(isopropyl)orthotitanat | mg | 220 | | 90 | 90 | 70 | | |
| Reaktionszeit | h | 6 | 8 | 6 | 10 | 17 | 10 | 10 |
| Reaktionstemperatur | °C | 220 | 220 | 220 | 220 | 220 | 220 | 220 |
| Reaktionsdruck | hPa | 600-400 | 600-300 | 600-400 | 1013-400 | 600-400 | 1013-400 | 1013-400 |
| | | | | | | | | |

| **Carbonsäureabzug** | | | | | | | | |
|---|---|---|---|---|---|---|---|---|
| Temperatur | °C | 120-226 | 105-200 | 120-198 | 140-200 | 100-220 | 120-210 | 120-220 |
| Druck | hPa | 1 | 1,2 | 2 | 3 | 2 | 3 | 3 |
| | | | | | | | | |

| **Wasserdampfdestillation** | | | | | | | | |
|---|---|---|---|---|---|---|---|---|
| Zeit | min | | 120 | | 105 | 105 | 105 | 105 |
| Temperatur | °C | | 180 | | 180 | 160 | 180 | 180 |
| | | | | | | | | |
| **Trocknung** | | | | | | | | |
| Zeit | min | | 30 | | 30 | 30 | 30 | 30 |
| Temperatur | °C | | 140 | | 140 | 140 | 140 | 140 |
| Druck | hPa | | 2 | | 2 | 2 | 2 | 2 |
| Polyol | | TEG | NPG | 1,3 BG | TCD | TMP | Pentaerythrit | Di-Pentaerythrit |

| **Gaschromatographische Analyse (%)** | | | | | | | | |
|---|---|---|---|---|---|---|---|---|
| Säure | | 0,1 | 0,1 | 0,1 | 0,3 | 0,1 | 0,1 | 0,1 |
| Halb-Ester | | 1,3 | 0,4 | 0,4 | 1,2 | 1,2 | 0,11 | 0,5 |
| Voll-Ester | | 98,4 | 99,1 | 99,2 | 98,0 | 98,5 | 99,2 | 98,8 |
| Nachlauf | | 0,2 | 0,4 | 0,3 | 0,5 | 0,2 | 0,6 | 0,6 |
| | | | | | | | | |

| **Kennzahlen** | | | | | | | | |
|---|---|---|---|---|---|---|---|---|
| VZ DIN/ENISO 3681 | mg KOH/ g | 258 | 284-287 | | 224-227 | 302-303 | 377 - 387 | 368 - 370 |
| V₂₀ ASTM D 445 | mm²/s | 21,00 | 19,53-19,75 | 15,30 | 134,30 | 64,16 | 58,19 | 170,8-172,6 |
| V₄₀ | mm²/s | | 9,41-9,43 | 7,77 | 45,2 | 24,96 | 24,28 | 62,3-62,5 |
| V₅₀ | mm²/s | 7,72 | 7,01 | 5,92 | 29,30 | 16,82 | | 41,39 |
| d^{20/4} DIN 51757, Verf.D/ASTM D 4052 | g/cm³ | 0,9638 | 0,915-0,917 | 0,918 | 0,976 | 0,9415 | 0,9814 | 0,998 |
| d^{40/4} | g/cm³ | | | 0,903 | 0,962 | 0,927 | 0,9664 | 0,983-0,984 |
| d^{50/4} | g/cm3 | 0,9383 | 0,895 | 0,896 | 0,955 | 0,9198 | | 0,977 |
| n_{20/D} DIN 51 423-2/ASTM D 1747 | | 1,4488 | 1,445 | 1,443 | 1,476 | 1,453 | 1,453 | 1,457-1,458 |
| Erstarrungspunkt | °C | -69 | -71 | -75 | -51 | -59 | -63 | -53 |
| Flammpunkt ISO2719 | °C | >130 | >150 | >175 | >130 | >135 | >135 | >175 |

| | | | | | | | | |
|---|---|---|---|---|---|---|---|---|
| TEG - Triethylenglykol; NPG - Neopentylglykol; TMP - Trimethylolpropan; 1,3 BG - 1,3-Butandiol; TCD - 3(4);8(9)-Bishydroxymethyltricyclo[5.2.1.0²⁶]decan | | | | | | | | |

## Patentansprüche

1. Verfahren zur Herstellung von Carbonsäureestern eines Gemisches aus strukturverzweigten C9-Monocarbonsäuren ausgehend von 2-Ethylhexanol, **dadurch gekennzeichnet, dass** man
(a) 2-Ethylhexanol in Gegenwart eines Katalysators zu einem Octengemisch dehydratisiert;
(b) das nach Schritt a) erhaltene Octengemisch in Gegenwart einer Übergangsmetallverbindung der Gruppe VIII des Periodensystems der Elemente mit Kohlenmonoxid und Wasserstoff zu einem Gemisch isomerer Isononanale umsetzt;
(c) das nach Schritt b) erhaltene Gemisch isomerer Isononanale zu einem Gemisch aus strukturverzweigten C9-Monocarbonsäuren oxidiert; und
(d) das nach Schritt c) erhaltene Gemisch aus strukturverzweigten C9-Monocarbonsäuren mit Alkoholen zu Carbonsäureestern umsetzt.

2. Verfahren gemäß Anspruch 1, **dadurch gekennzeichnet, dass** man in Schritt a) als Katalysator Aluminiumoxid, Nickel niedergeschlagen auf Aluminiumoxid, oder Phosphorsäure niedergeschlagen auf Siliziumdioxid oder Aluminiumoxid verwendet.

3. Verfahren gemäß Anspruch 1 oder 2, **dadurch gekennzeichnet, dass** man in Schritt a) 2-Ethylhexanol in der Gasphase dehydratisiert.

4. Verfahren gemäß einem oder mehreren der Ansprüche 1 bis 3, **dadurch gekennzeichnet, dass** man in Schritt b) als Übergangsmetallverbindung der Gruppe VIII des Periodensystems der Elemente eine Kobalt- oder Rhodiumverbindung verwendet.

5. Verfahren gemäß einem oder mehreren der Ansprüche 1 bis 4, **dadurch gekennzeichnet, dass** die Umsetzung gemäß Schritt b) in Abwesenheit von komplexbildenden Organoelementverbindungen durchgeführt wird.

6. Verfahren gemäß einem oder mehreren der Ansprüche 1 bis 5, **dadurch gekennzeichnet, dass** man das nach Schritt b) erhaltene Gemisch isomerer Isononanale destilliert.

7. Verfahren gemäß einem oder mehreren der Ansprüche 1 bis 6, **dadurch gekennzeichnet, dass** die Oxidation in Schritt c) in Anwesenheit von Alkali- oder Erdalkalimetallcarboxylaten erfolgt.

8. Verfahren gemäß Anspruch 7, **dadurch gekennzeichnet, dass** man als Alkali- oder Erdalkalimetallcarboxylat Lithium-, Kalium-, Natrium-, Calcium- oder Bariumisononanoat verwendet.

9. Verfahren gemäß einem oder mehreren der Ansprüche 1 bis 8, **dadurch gekennzeichnet, dass** man in Schritt c) das Gemisch isomerer Isononanale in der Flüssigphase oxidiert.

10. Verfahren gemäß einem oder mehreren der Ansprüche 1 bis 9, **dadurch gekennzeichnet, dass** man in Schritt c) das Gemisch isomerer Isononanale mit Sauerstoff oder sauerstoffenthaltenden Gasen zu dem Gemisch aus struktuverzweigten C9-Monocarbonsäuren oxidiert.

11. Verfahren gemäß einem oder mehreren der Ansprüche 1 bis 10, **dadurch gekennzeichnet, dass** man in Schritt d) die Umsetzung in Gegenwart eines Adsorbens durchführt.

12. Verfahren gemäß einem oder mehreren der Ansprüche 1 bis 11, **dadurch gekennzeichnet, dass** man in Schritt d) die Umsetzung in Gegenwart eines Katalysators durchführt.

13. Verfahren gemäß einem oder mehreren der Ansprüche 1 bis 12, **dadurch gekennzeichnet, dass** man in Schritt d) Alkohole der allgemeinen Formel (I) umsetzt
R(OH)ₙ (I)
in der R einen aliphatischen, cycloaliphatischen oder aromatischen Kohlenwasserstoffrest mit 1 bis 20, vorzugsweise 2 bis 10 Kohlenstoffatomen und n eine ganze Zahl von 1 bis 8, vorzugsweise 1, 2, 3, 4, 5 oder 6 ist.

14. Verfahren gemäß einem oder mehreren der Ansprüche 1 bis 12, **dadurch gekennzeichnet, dass** man in Schritt d) Alkohole der allgemeinen Formel (II) umsetzt
H-(-O-[-CR¹R²-]ₘ-)ₒ-OH (II)
in der R¹ und R² unabhängig voneinander Wasserstoff, einen Alkylrest mit 1 bis 5 Kohlenstoffatomen, vorzugsweise Methyl, Ethyl oder Propyl, oder einen Hydroxyalkylrest mit 1 bis 5 Kohlenstoffatomen, vorzugsweise der Hydroxymethylrest, m eine Zahl von 1 bis 10, vorzugsweise 1 bis 8 und insbesondere 1, 2, 3 oder 4, o eine ganze Zahl von 2 bis 15, vorzugsweise 2 bis 8 und insbesondere 2, 3, 4 oder 5 bedeuten.

15. Verfahren gemäß einem oder mehreren der Ansprüche 1 bis 12, **dadurch gekennzeichnet, dass** man in Schritt d) als Alkohol Glycidylalkohol einsetzt.

16. Carbonsäureester des Triethylenglykols, Neopentylglykols oder 1,3-Butandiols und eines Gemisches aus strukturverzweigten C9-Monocarbonsäuren enthaltend 4-Methyloctansäure, 6-Methyloctansäure, 2,5-Dimethylheptansäure, 2,3-Dimethylheptansäure, 3-Ethylheptansäure, 2-Ethylheptansäure und 2-Ethyl-4-methylhexansäure in Summe mindestens 80 mol-%, bezogen auf den Gesamtgehalt an strukturverzweigten C9-Monocarbonsäuren.

17. Verwendung der Carbonsäureester gemäß Anspruch 16 als Weichmacher für thermoplastische Kunststoffe.

18. Verwendung der Carbonsäureester gemäß Anspruch 16 als Schmiermittel.

## Claims

1. Process for preparing carboxylic esters of a mixture of structurally branched C9 monocarboxylic acids proceeding from 2-ethylhexanol, **characterized in that**
(a) 2-ethylhexanol is dehydrated to an octene mixture in the presence of a catalyst;
(b) the octene mixture obtained in step a) is reacted in the presence of a transition metal compound of group VIII of the periodic table of the elements with carbon monoxide and hydrogen to give a mixture of isomeric isononanals;
(c) the mixture of isomeric isononanals obtained in step b) is oxidized to a mixture of structurally branched C9 monocarboxylic acids; and
(d) the mixture of structurally branched C9 monocarboxylic acids obtained in step c) is reacted with alcohols to give carboxylic esters.

2. Process according to Claim 1, **characterized in that** the catalyst used in step a) is alumina, nickel precipitated on alumina, or phosphoric acid precipitated on silica or alumina.

3. Process according to Claim 1 or 2, **characterized in that** 2-ethylhexanol is dehydrated in the gas phase in step a).

4. Process according to one or more of Claims 1 to 3, **characterized in that** the transition metal compound of group VIII of the periodic table of the elements used in step b) is a cobalt or rhodium compound.

5. Process according to one or more of Claims 1 to 4, **characterized in that** the reaction in step b) is performed in the absence of complex-forming organoelemental compounds.

6. Process according to one or more of Claims 1 to 5, **characterized in that** the mixture of isomeric isononanals obtained in step b) is distilled.

7. Process according to one or more of Claims 1 to 6, **characterized in that** the oxidation in step c) is effected in the presence of alkali metal or alkaline earth metal carboxylates.

8. Process according to Claim 7, **characterized in that** the alkali metal or alkaline earth metal carboxylate used is lithium isononanoate, potassium isononanoate, sodium isononanoate, calcium isononanoate or barium isononanoate.

9. Process according to one or more of Claims 1 to 8, **characterized in that** the mixture of isomeric isononanals is oxidized in the liquid phase in step c).

10. Process according to one or more of Claims 1 to 9, **characterized in that** the mixture of isomeric isononanals is oxidized in step c) with oxygen or oxygen-containing gases to the mixture of structurally branched C9 monocarboxylic acids.

11. Process according to one or more of Claims 1 to 10, **characterized in that** the reaction in step d) is performed in the presence of an adsorbent.

12. Process according to one or more of Claims 1 to 11, **characterized in that** the reaction in step d) is performed in the presence of a catalyst.

13. Process according to one or more of Claims 1 to 12, **characterized in that** the alcohols converted in step d) are of the general formula (I)
R(OH)ₙ (I)
in which R is an aliphatic, cycloaliphatic or aromatic hydrocarbyl radical having 1 to 20 and preferably 2 to 10 carbon atoms, and n is an integer from 1 to 8, preferably 1, 2, 3, 4, 5 or 6.

14. Process according to one or more of Claims 1 to 12, **characterized in that** the alcohols converted in step d) are of the general formula (II)
H-(-O-[-CR¹R²-]ₘ-)ₒ-OH (II)
in which R¹ and R² are each independently hydrogen, an alkyl radical having 1 to 5 carbon atoms, preferably methyl, ethyl or propyl, or a hydroxyalkyl radical having 1 to 5 carbon atoms, preferably the hydroxymethyl radical, m is a number from 1 to 10, preferably 1 to 8 and especially 1, 2, 3 or 4, o is an integer from 2 to 15, preferably 2 to 8 and especially 2, 3, 4 or 5.

15. Process according to one or more of Claims 1 to 12, **characterized in that** the alcohol used in step d) is glycidyl alcohol.

16. Carboxylic ester of triethylene glycol, neopentyl glycol or 1,3-butanediol and of a mixture of structurally branched C9 monocarboxylic acids comprising 4-methyloctanoic acid, 6-methyloctanoic acid, 2,5-dimethylheptanoic acid, 2,3-dimethylheptanoic acid, 3-ethylheptanoic acid, 2-ethylheptanoic acid and 2-ethyl-4-methylhexanoic acid in a total amount of at least 80 mol%, based on the total content of structurally branched C9 monocarboxylic acids.

17. Use of the carboxylic esters according to Claim 16 as plasticizers for thermoplastic polymers.

18. Use of the carboxylic esters according to Claim 16 as lubricants.

## Revendications

1. Procédé de fabrication d'esters d'acides carboxyliques d'un mélange d'acides monocarboxyliques en C9 à structure ramifiée à partir de 2-éthylhexanol, **caractérisé en ce que**
(a) du 2-éthylhexanol est déshydraté en présence d'un catalyseur pour obtenir un mélange d'octènes ;
(b) le mélange d'octènes obtenu à l'étape a) est mis en réaction en présence d'un composé de métal de transition du groupe VIII du tableau périodique des éléments avec du monoxyde de carbone et de l'hydrogène pour obtenir un mélange d'isononanals isomères ;
(c) le mélange d'isononanals isomères obtenu à l'étape b) est oxydé en un mélange d'acides monocarboxyliques en C9 à structure ramifiée ; et
(d) le mélange d'acides monocarboxyliques en C9 à structure ramifiée obtenu en c) est mis en réaction avec des alcools pour former des esters d'acides carboxyliques.

2. Procédé selon la revendication 1, **caractérisé en ce que** de l'oxyde d'aluminium, du nickel précipité sur de l'oxyde d'aluminium ou de l'acide phosphorique précipité sur du dioxyde de silicium ou de l'oxyde d'aluminium est utilisé en tant que catalyseur à l'étape a).

3. Procédé selon la revendication 1 ou 2, **caractérisé en ce qu'**à l'étape a), le 2-éthylhexanol est déshydraté en phase gazeuse.

4. Procédé selon une ou plusieurs des revendications 1 à 3, **caractérisé en ce qu'**à l'étape b), un composé de cobalt ou de rhodium est utilisé en tant que composé de métal de transition du groupe VIII du tableau périodique des éléments.

5. Procédé selon une ou plusieurs des revendications 1 à 4, **caractérisé en ce que** la réaction selon l'étape b) est réalisée en l'absence de composés d'organoéléments complexants.

6. Procédé selon une ou plusieurs des revendications 1 à 5, **caractérisé en ce que** le mélange d'isononanals isomères obtenu à l'étape b) est distillé.

7. Procédé selon une ou plusieurs des revendications 1 à 6, **caractérisé en ce que** l'oxydation à l'étape c) a lieu en présence de carboxylates de métaux alcalins ou alcalino-terreux.

8. Procédé selon la revendication 7, caractérisé en ce ce que de l'isononanoate de lithium, potassium, sodium, calcium ou baryum est utilisé en tant que carboxylate de métal alcalin ou alcalino-terreux.

9. Procédé selon une ou plusieurs des revendications 1 à 8, **caractérisé en ce qu'**à l'étape c), le mélange d'isononanals isomères est oxydé en phase liquide.

10. Procédé selon une ou plusieurs des revendications 1 à 9, **caractérisé en ce qu'**à l'étape c), le mélange d'isononanals isomères est oxydé avec de l'oxygène ou des gaz contenant de l'oxygène pour obtenir le mélange d'acides monocarboxyliques en C9 à structure ramifiée.

11. Procédé selon une ou plusieurs des revendications 1 à 10, **caractérisé en ce qu'** à l'étape d), la réaction est réalisée en présence d'un adsorbant.

12. Procédé selon une ou plusieurs des revendications 1 à 11, **caractérisé en ce qu'** à l'étape d), la réaction est réalisée en présence d'un catalyseur.

13. Procédé selon une ou plusieurs des revendications 1 à 12, **caractérisé en ce qu'** à l'étape d), des alcools de formule générale (I) sont mis en réaction
R(OH)ₙ (I)
dans laquelle R est un radical hydrocarboné aliphatique, cycloaliphatique ou aromatique de 1 à 20, de préférence 2 à 10 atomes de carbone, et n est un nombre entier de 1 à 8, de préférence 1, 2, 3, 4, 5 ou 6.

14. Procédé selon une ou plusieurs des revendications 1 à 12, **caractérisé en ce qu'** à l'étape d), des alcools de formule générale (II) sont mis en réaction
H-(-O-[-CR¹R²-]ₘ-)ₒ-OH (II)
dans laquelle R¹ et R² signifient indépendamment l'un de l'autre l'hydrogène, un radical alkyle de 1 à 5 atomes de carbone, de préférence méthyle, éthyle ou propyle, ou un radical hydroxyalkyle de 1 à 5 atomes de carbone, de préférence le radical hydroxyméthyle, m signifie un nombre de 1 à 10, de préférence de 1 à 8, et notamment 1, 2, 3 ou 4, o signifie un nombre entier de 2 à 15, de préférence de 2 à 8, et notamment 2, 3, 4 ou 5.

15. Procédé selon une ou plusieurs des revendications 1 à 12, **caractérisé en ce qu'** à l'étape d), de l'alcool glycidylique est utilisé en tant qu'alcool.

16. Esters d'acides carboxyliques de triéthylène glycol, de néopentylglycol ou de 1,3-butanediol et d'un mélange d'acides monocarboxyliques en C9 à structure ramifiée contenant de l'acide 4-méthyloctanoïque, de l'acide 6-méthyloctanoïque, de l'acide 2,5-diméthylheptanoïque, de l'acide 2,3-diméthylheptanoïque, de l'acide 3-éthylheptanoïque, de l'acide 2-éthylheptanoïque et de l'acide 2-éthyl-4-méthylhexanoïque au total à hauteur d'au moins 80 % en moles, par rapport à la teneur totale en acides monocarboxyliques en C9 à structure ramifiée.

17. Utilisation des esters d'acides carboxyliques selon la revendication 16 en tant que plastifiant pour des plastiques thermoplastiques.

18. Utilisation des esters d'acides carboxyliques selon la revendication 16 en tant que lubrifiant.
